# EUROPEAN PATENT APPLICATION

(11) **EP 4 640 770 A1**
(43) Date of publication of application: **29.10.2025**
(21) Application number: 23906741.6
(22) Date of filing: 07.12.2023
(51) Int. Cl.: C09B 67/02, A23L 5/43, A23L 5/44, A61K 8/19, A61K 8/24, A61K 8/31, A61K 8/49, A61K 47/02, A61K 47/06, A61K 47/22, C09B 61/00, C09C 1/02, C09C 1/42, C09C 3/08

(54) **WATER-INSOLUBLE PIGMENT COMPOSITION**

(30) Priority: 22.12.2022 JP 2022205433
(71) Applicant: DIC Corporation, Tokyo 174-8520 (JP)
(72) Inventor: SEKIKAWA Hiroshi, Kamisu-shi, Ibaraki 314-0193 (JP); TANAKA Masaaki, Kamisu-shi, Ibaraki 314-0193 (JP); YASUI Kengo, Kamisu-shi, Ibaraki 314-0193 (JP)
(74) Representative: TBK
(86) International application number: PCT/JP2023/043752
(87) International publication number: WO 2024/135375

(57) **Abstract**

An object of the present invention is to provide a water-insoluble pigment composition produced by compositing a natural pigment with at least one selected from hydroxyapatite and a clay mineral and provide a food, a cosmetic, a lipstick cosmetic, an eye cosmetic, a nail cosmetic, a base makeup cosmetic, a pharmaceutical, a coating material for agricultural chemicals, a printing marker, a stationery product, a writing tool, a printing ink, an inkjet ink, a metal ink, a paint, a plastic coloring agent, a color toner, a fluorescent labeling agent, a fluorescent probe, or a chemical sensor, each including the water-insoluble pigment composition. The inventors have found that a water-insoluble pigment composition is produced by compositing a natural pigment with hydroxyapatite or a clay mineral, and thus have accomplished the present invention.

## Description

### Technical Field

The present invention relates to a pigment composition.

### Background Art

As natural pigments, various types of red pigments, yellow pigments, and blue pigments are present. In recent years, synthetic coloring agents have been regarded as problematic in terms of carcinogenicity or the like, and greater expectations have been placed on natural pigments, which are considered to have a higher level of safety.

In addition, business activities that strongly focuses on sustainability have attracted attention, and environmentally friendly colorants have been sought. Under these circumstances, there is a problem that many types of natural pigments dissolve easily in water, and therefore, when natural pigments are used in cosmetics or food pigments, the natural pigments dissolve in water to cause color fading. In addition, when natural pigments are used in flexographic printing, problems such as migration and plate staining arise. Therefore, under the current circumstances, natural pigments are used only in very limited applications.

The inventors investigated the literatures describing how to solve the problems of dissolution of natural pigments in water and color fading, that is, describing the improvement of water solubility, and found a description of a method for extracting natural pigments and a description of orally administered cosmetics, dairy products, and pet foods that are produced using extracts of natural pigments (PTL 1). There is another description of dry-blending carotenoid pigments with sodium tartrate, alum, and sodium carbonate and dissolving the resulting mixed powder in water to obtain an aqueous solution for the purpose of hue stabilization (PTL 2). There is another description of a powder composition including carotenoid, an oil component, and sugar (PTL 3).

However, these literatures do not offer a solution for a problem about the water solubility of natural pigments. There is no example in which the water solubility of natural pigments can be improved, and the solution has been desired.

### Citation List

### Patent Literature

PTL 1: Japanese Unexamined Patent Application Publication (Translation of PCT Application) No. 2009-508505
PTL 2: Japanese Examined Patent Application Publication No. S59-050264
PTL 3: Japanese Unexamined Patent Application Publication No. 2009-185023

### Summary of Invention

### Technical Problem

An object of the present invention is to provide a water-insoluble pigment composition and provide a food, a cosmetic, a lipstick cosmetic, an eye cosmetic, a nail cosmetic, a base makeup cosmetic, a pharmaceutical, a coating material for agricultural chemicals, a printing marker, a stationery product, a writing tool, a printing ink, an inkjet ink, a metal ink, a paint, a plastic coloring agent, a color toner, a fluorescent labeling agent, a fluorescent probe, or a chemical sensor, including the water-insoluble pigment composition.

### Solution to Problem

The inventors have conducted extensive studies to solve the above-mentioned problem, and, as a result, found that a water-insoluble pigment composition is produced by compositing a natural pigment with hydroxyapatite or a clay mineral, and thus have accomplished the present invention.

In other words, the present invention encompasses the following aspects.
[1] A water-insoluble pigment composition, produced by compositing a natural pigment with at least one selected from hydroxyapatite and a clay mineral.
[2] The water-insoluble pigment composition according to [1], wherein the composition mass ratio of the natural pigment to the at least one selected from hydroxyapatite and the clay mineral is the natural pigment : hydroxyapatite or the clay mineral = **0.1:99.9** to 90:10.
[3] The water-insoluble pigment composition according to [1] or [2], wherein the clay mineral is at least one selected from bentonite, hectorite, and smectite.
[4] The water-insoluble pigment composition according to any one of [1] to [3], wherein the clay mineral is at least one selected from organically-modified bentonite, organically-modified hectorite, and organically-modified smectite.
[5] The water-insoluble pigment composition according to any one of [1] to [4], wherein the natural pigment is at least one selected from a carotenoid pigment, a porphyrin pigment, a flavonoid pigment, and a pigment protein.
[6] The water-insoluble pigment composition according to any one of [1] to [4], wherein the natural pigment is at least one selected from an annatto pigment, a chlorophyll pigment, a safflower yellow pigment, and a phycocyanin pigment.
[7] A food, a cosmetic, a lipstick cosmetic, an eye cosmetic, a nail cosmetic, a base makeup cosmetic, a pharmaceutical, a coating material for agricultural chemicals, a printing marker, a stationery product, a writing tool, a printing ink, an inkjet ink, a metal ink, a paint, a plastic coloring agent, a color toner, a fluorescent labeling agent, a fluorescent probe, or a chemical sensor, including the water-insoluble pigment composition according to any one of [1] to [6]. Advantageous Effects of Invention

According to the present invention, a water-insoluble pigment composition can be provided.

### Description of Embodiments

Hereinafter, a water-insoluble pigment composition according to the present invention will be described in detail, but the following descriptions about constituents are merely examples as embodiments of the present invention, and the present invention is not limited by the descriptions.

### (Natural pigment)

The natural pigment that can be used in the present invention are any natural pigment, such as red cabbage pigment, red radish pigment, annatto pigment, squid ink pigment, turmeric pigment, cacao pigment, carotene pigment, carotenoid pigment, gardenia red pigment, gardenia blue pigment, gardenia yellow pigment, chlorophyll pigment, kaoliang pigment, cochineal pigment, saffron pigment, perilla pigment, rosewood pigment, spirulina pigment, phycocyanin pigment, onion pigment, tamarind pigment, clitoria ternatea pigment, capsicum pigment, tomato pigment, hibiscus pigment, beet red pigment, grape skin pigment, Haematococcus pigment, Monascus purpureus pigment, safflower red pigment, safflower yellow pigment, berry pigment, marigold pigment, purple potato pigment, purple corn pigment, purple yam pigment, caramel pigment, and plant charcoal pigment. What is biosynthesized, enzymatically synthesized, or chemically synthesized from the above-mentioned pigments can be used as well.

Among them, carotenoid pigments, porphyrin pigments, flavonoid pigments, and pigment proteins can be preferably used in the present invention.

Examples of known carotenoid pigments include red pigments extracted from carrots, tomatoes, and peppers, and yellow pigments extracted from citrus fruits, gardenias, annattos, and marigolds. Green leafy vegetables contain carotenoid pigments in large amounts.

Carotenoid is a substance belonging to tetraterpenes of terpene compounds. Terpenes are important compounds for plants and 20,000 or more types of terpene compounds are known. Examples of known carotenoid pigments include: hydrocarbon pigments, such as β-carotene and lycopene; xanthophyll pigments, such as astaxanthin, capsanthin, and lutein; bixin, norbixin, and crocin.

Porphyrin pigments have four pyrrole rings, and metal complexes thereof are included in, for example, chlorophyll, which plays a role in photoabsorption and photoelectron transfer in photosynthesis, or the heme of hemoglobin, which carries oxygen in the blood, and thus play an important role in the body. The porphyrin metal complexes have been widely used as photoelectronic functional materials, metal complex catalysts, and molecular conductive materials, and are known to change the peripheral substituent, the central metal, and the axial ligand of porphyrin and thereby exhibit a wide variety of functions.

Flavonoids are a large group of polyphenols. Flavonoids are a general name for components with a certain chemical structure and included in leaves, stems, and trunks of plants, and are substances produced by plants to protect the plants from ultraviolet rays and harmful insects, and are sources of pigments and bitter taste components. Flavonoids are classified into flavonols, flavones, catechins, flavanones, anthocyanins, and isoflavones, based on the structures.

Examples of flavonoid pigments include flavonols, anthocyanin pigments, which exhibit various color tones, chalcones, which develop a dark yellow color, and aurones.

### (Pigment protein)

Pigment protein is a general term for protein composited with a pigment in the natural state. Pigment protein is present in cells and body fluids of plants and animals and express various color tones and physiological functions, depending on a prosthetic group including a pigment. Hemoprotein is a composite of an iron-porphyrin complex salt and protein and has a protein-to-heme binding ratio of, for example, 1:1, 1:2, or 1:4. Hemoprotein is widely present in nature and examples thereof include hemoglobin, myoglobin, cytochrome, catalase, and peroxidase.

A metal complex compound is a composite of metal complex ions and protein and examples thereof include copper proteins such as hemocyanin and iron proteins such as ferritin. Ferritin is present in the spleen, tunica mucosa intestini tenuis, and liver, for example, and is thought to be involved in iron storage in vivo and iron absorption during digestion.

Phycopigment protein is a composite of a pyrrole derivative and protein, and examples thereof include phycoerythrin, which gives red color to red algae plants, and phycocyanin, which gives indigo blue color to cyanobacteria plants. Phycopigment protein is present in chloroplasts along with chlorophyll and carotenoid, and thought to be accessory pigments for photosynthesis.

Flavin protein has flavin mononucleotide or flavin adenine dinucleotide as a prosthetic group. All types of flavin protein act as oxidoreductase and are also called flavoenzymes. Examples of flavin protein include amino acid oxidase and xanthine oxidase.

Carotenoid protein is a composite of carotenoid and protein. Examples of carotenoid protein include a composite of Vitamin A and protein, such as rhodopsin.

### (Phycocyanin)

Phycocyanin is the most preferred pigment protein to be used in the present invention because phycocyanin develops bright blue color. Phycocyanin is a pigment protein and contains phycocyanobilin as a chromophore. Phycocyanin has a structure in which phycocyanobilin is bound to a protein.

Examples of phycocyanin according to the present invention include algae phycocyanin, such as cyanobacteria phycocyanin, red algae phycocyanin, and cryptophyte phycocyanin. Among them, cyanobacteria phycocyanin is preferred because cyanobacteria phycocyanin can be collected in large amounts.

Examples of cyanobacteria include cyanobacteria of the genus Spirulina, the genus Arthrospira, the genus Aphanizomenon, the genus Fisherella, the genus Anabaena, the genus Nostoc, the genus Synechocystis, the genus Synechococcus, the genus Tolypothrix, the genus Aphanothece, the genus Mastigoclaus, and the genus Pleurocapsa. Among them, cyanobacteria of the genus Spirulina and the genus Arthrospira, which have been produced on an industrial scale and the safety of which has been confirmed, are preferable, and cyanobacteria of the genus Spirulina are more preferable.

As a raw material for preparing the phycocyanin, fresh cyanobacteria or dried cyanobacteria may be used. A dried cyanobacteria product may be obtained by drying fresh cyanobacteria according to the usual method, or a commercially available dried cyanobacteria product can be used.

The phycocyanin can be obtained, for example, by suspending cyanobacteria in water or a buffer solution such as a phosphate buffer solution or a citrate buffer solution, and extracting phycocyanin from the cyanobacteria.

A method for extracting the phycocyanin is not particularly limited, and a commonly known method can be used.

Examples of a preferred embodiment of the extraction method include an extraction method described in Japanese Unexamined Patent Application Publication No. 2006-230272. Specific examples of the extraction method include an extraction method (i) described below. Using the extraction method (i), high-purity phycocyanin assuming a vivid color tone can be obtained.

### <Extraction Method (i)>

The extraction method (i) includes:
a first step of obtaining a liquid extract in which phycocyanin in cyanobacteria is extracted into an aqueous suspension;
a second step of allowing a calcium salt to react with a phosphate in the liquid extract to produce calcium phosphate and causing the calcium phosphate to adsorb phycocyanin contaminants, thereby obtaining an adsorbate; and
a third step of removing a cyanobacterial residue and the adsorbate from the liquid extract.

Furthermore, the above-described extraction method (i) is more preferably an extraction method (ii) described below.

### <Extraction Method (ii)>

The extraction method (ii) includes:
a first step of obtaining a liquid extract in which phycocyanin in cyanobacteria is extracted into an aqueous suspension;
a second step of allowing a calcium salt to react with a phosphate in the liquid extract to produce calcium phosphate and causing the calcium phosphate to adsorb phycocyanin contaminants, thereby obtaining an adsorbate;
a third step of removing a cyanobacterial residue and the adsorbate from the liquid extract; and
a step of, prior to the third step, adding a chelating agent to the liquid extract.

The phycocyanin to be used in the present invention was obtained from a commercial product, LINABLUE G1 (manufactured by DIC LIFETEC Co., Ltd., trehalose: 55%, Spirulina extract: 40%, trisodium citrate: 5%), in which phycocyanin was mixed with a stabilizing agent. As described in Japanese Unexamined Patent Application Publication No. H11-299450, trehalose is used to increase thermal stability and citric acid is used as a pH adjuster. Note that phycocyanin pigment is contained as a main component in the spirulina extract.

In the present invention, one type of natural pigment may be used alone or several types of natural pigments may be used in combination. Depending on the application, natural pigments may be pre-mixed and composited with a carrier substance to achieve a desired hue, or water-insoluble pigment compositions each obtained by compositing a single natural pigment with a carrier substance may be mixed to achieve the desired hue.

In the present invention, one type of natural pigment may be used alone or several types of natural pigments may be used in combination. Depending on the application, natural pigments may be pre-mixed and composited with hydroxyapatite or a clay mineral to achieve a desired hue, or water-insoluble pigment compositions each obtained by compositing a single natural pigment with hydroxyapatite or a clay mineral may be mixed to achieve the desired hue.

### (Hydroxyapatite)

Hydroxyapatite to be used in the present invention is one kind of calcium phosphate and is a main component of teeth and bones. Hydroxyapatite, which is present in nature as a mineral and a constituent of living organisms, has been widely used as a synthetic component for medical devices and dental materials (for example, a coating for implants, a material for bone formation, and an artificial tooth root) because of its high biocompatibility. In addition, hydroxyapatite has the unique property of retaining its crystal structure even when some ions in a crystal are substituted. Hence, subtly controlling the ions in the crystal and the shape of the crystal allows hydroxyapatite to have various functions, such as a selective adsorption function of proteins and a catalytic function, and thus hydroxyapatite has been widely used in the chemical industry.

Hydroxyapatite having lower crystallinity tends to have a larger specific surface area and a higher adsorption capacity. From this viewpoint, the hydroxyapatite to be used in the present invention is preferably low-crystalline hydroxyapatite having a Ca/P (molar ratio) of from 1.4 to 1.8, which serves as a carrier substance. Similarly, from the viewpoint of the amount of adsorption, the hydroxyapatite preferably has a specific surface area of 10 m²/g or larger (more preferably 40 m²/g or larger), which is measured by a nitrogen gas adsorption method. The amount of adsorption tends to increase as the bulk of hydroxyapatite increases, and therefore, the hydroxyapatite preferably has a bulk of 500 mL/100 g or more (more preferably 900 mL/100 g or more). The hydroxyapatite preferably has an average particle size of 0.1 to 40 µm, and more preferably 10 to 30 µm.

### (Clay mineral)

Clay minerals are minerals constituting clay and include a layered silicate mineral (a phyllosilicate mineral) as a main component thereof. Clay minerals have a structure in which sheets each formed by coupling metal ions (for example, aluminum, sodium, or calcium ions) to silicic acid are stacked in layers. Water, metal ions, and, in some cases, even organic matter easily enter gaps between the sheets and are easily released, and therefore clay minerals have been used as a functional material, such as a humidity controller, an ion exchanger, or a catalyst, in various fields, including household goods. Clay minerals have been also used as a raw material of ceramic materials and pottery.

Examples of a clay mineral to be used in the present invention include bentonite, hectorite, smectite, kaolinite, montmorillonite, sericite, illite, glauconite, chlorite, talc, and zeolite.

Among them, bentonite, hectorite, and smectite are particularly desirable as the clay mineral in the present invention because of their great affinity for pigments, high water-insolubility, and excellent performance of color development.

The crystalline phases of bentonite, hectorite, and smectite are negatively charged. Cations that compensate for electric charge polarization are held between the layers of the sheet-like structure, and the area of a layer surface relative to the sheet thickness is large.

Organic modification is a treatment to insert an organic compound between layers of a clay mineral or to cause an organic compound to be adsorbed onto a surface of the clay mineral. In the present invention, the organic compound to be used for the organic modification is not particularly limited, and examples thereof include an organic compound having a cationic group, and especially preferred examples thereof include an organic compound having an ammonium group. The molecular weight of the above-mentioned organic compound is not particularly limited, but the organic compound has a carbon number of preferably 10 to 500 and more preferably 20 to 100. Note that examples of an agent for the organic modification include not only the organic compound but also a salt thereof. Specific examples of the organic compound include:
quaternary ammonium, such as dialkyl dimethyl ammonium, alkyl trimethyl ammonium, alkyl dimethyl ammonium, and alkyl dimethyl benzyl ammonium; phosphonium; sulfonium;
imidazolium; and pyridinium. Among them, quaternary ammonium is particularly preferred. The quaternary ammonium is not limited to a particular one, but didecyldimethylammonium, dimethyldistearylammonium, and cetyltrimethylammonium are preferably used.

The surfaces of organically-modified bentonite, organically-modified hectorite, and organically-modified smectite that have been subjected to the organic-modification treatment are hydrophobic and therefore have a stronger hydrophobic interaction with a hydrophobic site present in the chemical structure of a natural pigment, such as annatto pigment, chlorophyll pigment, safflower yellow pigment, or phycocyanin pigment, and hence the pigment is more easily adsorbed onto the surfaces. From the viewpoint of ease of pigment adsorption and the amount of the pigment adsorbed, the organically-modified clay mineral is preferably used in the present invention.

The particle shape of the clay mineral to be used is such that the secondary particle size is preferably 0.1 to 100 µm and more preferably 5 to 50 µm.

### (Water-insoluble Pigment Composition)

Most of natural pigments to be used in the present invention are naturally derived and basically in the form of dyes and accordingly water-soluble. However, in the present invention, the inventors have found that the natural pigment is firmly composited with hydroxyapatite or the clay mineral by coating, impregnation, or adsorption, whereby the resulting composite is insoluble in water as a water-insoluble pigment composition. Compositing mentioned herein is not limited to coating, impregnation, or adsorption, and the resulting composite is not merely a mixture, but is defined as what is formed in such a manner that a natural pigment interacts with hydroxyapatite or a clay mineral via physical or chemical adsorption to become water-insoluble.

With the water-insolubility achieved according to the present invention, the natural pigment has a tolerance enough to be used, as a coloring material equivalent to a common pigment, for applications to food, cosmetics, lipstick cosmetics, eye cosmetics, nail cosmetics, base makeup cosmetics, pharmaceuticals, coating materials for agricultural chemicals, printing markers, stationery products, writing tools, printing ink, inkjet ink, metal ink, paints, plastic coloring agents, color toners, fluorescent labeling agents, fluorescent probes, chemical sensors, or the likes. Furthermore, with the insolubility, improvement of properties such as heat resistance and light resistance can be expected. Note that the applications of the water-insoluble pigment composition according to the present invention are not limited to the above-mentioned applications.

For the water-insoluble pigment composition according to the present invention, the composition mass ratio of the natural pigment to hydroxyapatite or the clay mineral can be set to the natural pigment : hydroxyapatite or the clay mineral = 0.1:99.9 to 90:10. The composition ratio is preferably the natural pigment : hydroxyapatite or the clay mineral = 1:99 to 70:30 by mass.

The average particle size of the water-insoluble pigment composition is preferably within a range of 0.1 µm to 100 µm, more preferably within a range of 1 µm to 50 µm.

The content of an element derived from hydroxyapatite or the clay mineral used in the water-insoluble pigment composition according to the present invention is preferably 99% or lower, more preferably 90% or lower, and still more preferably 85% or lower, with respect to hydroxyapatite itself or the clay mineral itself.

### (Method for Producing Water-insoluble Pigment Composition)

As a method for producing the water-insoluble pigment composition according to the present invention, a method of mixing the natural pigment with hydroxyapatite or the clay mineral in a solvent is preferred because this method makes it possible to produce the most uniform water-insoluble pigment composition.

Examples of the method for producing the water-insoluble pigment composition by which the substances are mixed in a solvent include a method in which: 1) a liquid dispersion of hydroxyapatite or the clay mineral is prepared first. 2) On the other hand, the natural pigment is dissolved in water to prepare an aqueous solution. 3) Next, the above-described two types of the liquids are mixed to produce the water-insoluble pigment composition. Here, the aqueous solution of the natural pigment may be added to and mixed with the liquid dispersion of hydroxyapatite or the clay mineral, or alternatively the liquid dispersion of hydroxyapatite or the clay mineral may be added to and mixed with the aqueous solution of the natural pigment, or alternatively these two liquids may be mixed little by little to produce the water-insoluble pigment composition. The mixing may be performed at room temperature or may be performed with heating. From the viewpoint of the decomposition temperature of the natural pigment as a single substance, the mixing is performed preferably at 10°C to 60°C, and more preferably at 20°C to 50°C. 4) The pH of the mixed solution is adjusted and the natural pigment is composited with the carrier substance to produce the water-insoluble pigment composition.

By filtering and drying the obtained mixed solution, the water-insoluble pigment composition can be obtained. When the mixed solution is filtered through a filter such as a Nutsche filter, there is no coloration in a filtrate, whereby it can be confirmed that the natural pigment has been composited with hydroxyapatite or the clay mineral. Furthermore, when a wet cake of the water-insoluble pigment composition is repeatedly washed with water, similarly, the filtrate is clear and colorless, whereby it can be confirmed that the pigment component has not leaked out. The obtained water-containing wet cake of the water-insoluble pigment composition is dried at room temperature, or dried by heating, vacuum, vacuum-drying, or other means, whereby a dry water-insoluble pigment composition can be obtained. Any drying method and any dryer can be used for the drying as long as the method and the dryer are usual ones, and the method and the dryer are not limited to particular ones.

The water-insoluble pigment composition according to the present invention may be a water-insoluble pigment composition in the form of the above-mentioned water-containing wet cake or may be a dry water-insoluble pigment composition, and either one can be used properly, depending on the applications. When used in a water dispersion or ink, the wet cake can be used as it is, meanwhile, when used in a solvent dispersion system, the wet cake can be used after changed from a water base to a solvent base. The dry water-insoluble pigment composition can be used as it is, or of course can be re-dispersed in water, an organic solvent, a resin solution, or the like.

### (Stabilizer, Additive)

Of course other organic and inorganic pigments, dyes, and pigments can be mixed with the water-insoluble pigment composition according to the present invention at any ratio, and a desired and required hue can be achieved.

A stabilizer and an additive can be added to the water-insoluble pigment composition according to the present invention for the purpose of giving higher light-resistance and higher heat-resistance.

A stabilizer or an additive can be added to either the liquid dispersion of hydroxyapatite or the clay mineral or the aqueous solution of the natural pigment, or alternatively can be added to both the liquid dispersion of hydroxyapatite or the clay mineral and the aqueous solution of the natural pigment, or alternatively may be added to the produced water-insoluble pigment composition.

The water-insoluble pigment composition according to the present invention is mixed with another resin, a rubber, an additive, a pigment, a dye, or the like, as needed, so that the resulting mixture is adjusted to be a final product, such as a food, a cosmetic, a lipstick cosmetic, an eye cosmetic, a nail cosmetic, a base makeup cosmetic, a pharmaceutical, a coating material for agricultural chemicals, a printing marker, a stationery product, a writing tool, a printing ink, an inkjet ink, a metal ink, a paint, a plastic coloring agent, a color toner, a fluorescent labeling agent, a fluorescent probe, or a chemical sensor. Examples of the above-mentioned applications will be described below.

### (Cosmetics Applications)

The water-insoluble pigment composition according to the present invention can be used for a cosmetic. The water-insoluble pigment composition is not limited to be used in a particular cosmetic, but can be used in various types of cosmetics, such as cosmetics in a powder, liquid, gel, or solid form.

The cosmetic may be any type of cosmetics as long as the cosmetic can effectively perform functions. The cosmetic may be in the form of a lotion, a cream gel, a spray, or the like. Examples of the cosmetic include: skin care cosmetics, such as a facial cleanser, a make-up remover, a lotion, an essence, a pack, a protective emulsion, a protective cream, a whitening cosmetic, and a UV-protective cosmetic; make-up cosmetics, such as a foundation, white powder, a makeup base, a lipstick, eye makeup, a cheek rouge, and a nail enamel; hair care cosmetics, such as a shampoo, a hair rinse, a hair treatment, a hairdressing, a permanent wave agent, a hair dye, and a hair growth tonic; and body care cosmetics, such as a body wash cosmetic, a deodorant cosmetic, and a bath agent.

The amount of the water-insoluble pigment composition according to the present invention to be used in the cosmetic can be suitably set in accordance with the type of the cosmetic. The amount of the water-insoluble pigment composition contained in the cosmetic is usually within a range of 0.1% to 99% by mass, and is generally preferably within a range of 0.1% to 10% by mass. In contrast, in the make-up cosmetics with the objective of coloring, the amount of the water-insoluble pigment composition is preferably within a range of 5% to 80% by mass, more preferably within a range of 10% to 70% by mass, and most preferably within a range of 20% to 60% by mass. When the amount of the water-insoluble pigment composition according to the present invention contained in the cosmetic is within the above-mentioned range, a function such as coloring performance can be effectively performed and also a function required for the cosmetic can be retained.

Besides the water-insoluble pigment composition according to the present invention, the cosmetic may contain, depending on the type of the cosmetic, acceptable cosmetic ingredients, such as a carrier, a pigment, oil, sterol, amino acid, a moisturizer, powder, a coloring agent, a pH adjuster, a fragrance, essential oil, a cosmetic active ingredient, a vitamin, essential fatty acid, sphingolipid, a self-tanning agent, an excipient, a filler, an emulsifier, an antioxidant, a surfactant, a chelating agent, a gelling agent, a thickening agent, an emollient, a humectant, a moisturizer, a mineral, a viscosity modifying agent, a flow control agent, a keratolytic agent, retinoid, a hormonal compound, alpha-hydroxy acid, alpha-keto acid, an antimycobacterial agent, an antifungal agent, an antibacterial agent, an antiviral agent, a painkiller, an antiallergic agent, an antihistamine, an anti-inflammatory agent, an anti-irritant, an anti-tumor drug, an immune system boosting agent, an immune system inhibiting agent, an anti-acne agent, an anesthetic, a disinfectant, an insect repellent, a skin cooling compound, a skin barrier, a skin penetration enhancer, an exfoliant, a lubricant, an aromatic, a dye, a decoloring agent, a hypopigmenting agent, a preservative, a stabilizer, a pharmaceutical, a light stabilizer, and spherical powder.

One or more types of pigments other than the water-insoluble pigment composition may be added for the purpose of adjusting the color tone of cosmetics. Examples of the pigments include a white pigment, a colored pigment, an extender pigment, and a pearl pigment. Examples of the white pigment include titanium dioxide and zinc oxide. Examples of the colored pigment include: inorganic red pigments, such as iron oxide, iron hydroxide, and iron titanate; inorganic brown pigments, such as γ-iron oxide; inorganic yellow pigments, such as iron yellow oxide and ochre; inorganic black pigments, such as iron black oxide and carbon black; inorganic purple pigments, such as manganese violet and cobalt violet; inorganic green pigments, such as chromium hydroxide, chromium oxide, cobalt oxide, and cobalt titanate; inorganic blue pigments, such as iron blue and ultramarine blue; and tar pigments (such as Red 202, Red 204, Red 205, Red 220, Red 228, Yellow 401, Blue 404, Orange 203, and Orange 204); lakes formed from tar dyes (Red 3, Red 104, Red 106, Red 201, Red 226, Red 227, Red 230, Red 401, Red 505, Yellow 4, Yellow 5, Yellow 202, Yellow 203, Yellow 204, Blue 1, Blue 2, Blue 201, Green 3, Green 201, Green 204, Green 205, Orange 201, Orange 206, Orange 207, carminic acid, and laccaic acid), and synthetic resin powder obtained by compositing these types of powder. Examples of the extender pigment include: silicic acid, such as silica and hydrous silicic acid; silicates, such as aluminum silicate and magnesium silicate; clay minerals, such as talc, kaolin, bentonite, mica, and sericite; phosphate minerals, such as hydroxyapatite; metal oxides, such as aluminum oxide and magnesium oxide; carbonates of alkaline earth metals, such as light calcium carbonate, heavy calcium carbonate, light magnesium carbonate, and heavy magnesium carbonate; sulfates of alkaline earth metals, such as magnesium sulfate and barium sulfate (plate-shaped barium sulfate and butterfly-shaped barium sulfate); boron nitride; lauroyl lysine, organic powders, such as lauroyl lysine and metallic soap; plate-shaped synthetic powder, such as synthetic mica; resin powder, such as nylon beads, nylon powder, and silicone beads. Furthermore, the above-mentioned powder may be surface-treated with silicone, a fluorine compound, a silane coupling agent, silane, organic titanate, acylated lysine, a fatty acid, metal soap, an oil agent, or amino acid. Examples of the pearl pigment include titanium-dioxide-coated mica, titanium-dioxide-coated mica, bismuth oxychloride, titanium-dioxide-coated bismuth oxychloride, titanium-dioxide-coated talc, fish scale foil, and titanium-dioxide-coated colored mica.

The above-mentioned cosmetic can be produced by mixing the water-insoluble pigment composition according to the present invention with other cosmetic ingredients. The cosmetic containing the water-insoluble pigment composition according to the present invention can be used in the same way as for common cosmetics, depending on the type or the like of the cosmetic.

### (Ink and Paint Applications)

The water-insoluble pigment composition according to the present invention can be used for an ink or a paint. Note that the applications and composition of the ink or the paint will be described, but are not limiting. The water-insoluble pigment composition according to the present invention may be dispersed only in a thermoplastic resin, but can be dispersed in a printing ink vehicle or a paint vehicle that contains a thermoplastic resin as an essential ingredient.

As for the thermoplastic resin, for example, a polyester resin, a polyamide resin, a styrene resin, an acrylic resin, polyolefin, polyalkylene terephthalate, or a polyvinyl chloride resin can be used as a resin for dispersion.

For example, a planographic ink vehicle is produced from 20% to 50% (by mass) of a resin, such as a rosin-modified phenolic resin, a petroleum resin, or an alkyd resin; 0% to 30% (by mass) of animal fat or vegetable oil, such as linseed oil, tung oil, or soybean oil; 10% to 60% (by mass) of a solvent, such as n-paraffin, iso-paraffin, naphthene, α-olefin, or an aromatic; and a few percent (by mass) of an additive, such as a solubilizer or a gelling agent.

A gravure printing ink vehicle or a flexographic printing ink vehicle is produced, for example, from 10% to 50% (by mass) of at least one resin selected from rosins, a maleic acid resin, a polyamide resin, a vinyl resin, a cyclized rubber, a chlorinated rubber, an ethylene-vinyl acetate copolymer resin, a urethane resin, a polyester resin, an alkyd resin, nitrocellulose, cellulose acetate, and the like; and 30% to 80% (by mass) of a solvent, such as alcohols, toluene, n-hexane, ethyl acetate, cellosolve, or butyl cellosolve acetate.

A paint vehicle is produced from 20% to 80% (by mass) of a resin, such as an alkyd resin, an epoxy resin, an acrylic resin, a polyurethane resin, a polyester resin, a melamine resin, a urea resin, or a water-soluble resin; and 10% to 60% (by mass) of a solvent, such as hydrocarbons, alcohols, ketones, or water.

### (Plastic Applications)

The water-insoluble pigment composition according to the present invention can be used for plastic coloring applications. To obtain a colored plastic molded product, a thermoplastic resin (plastic) for thermoforming such as injection molding or press molding, for example, polyolefin, such as polyethylene or polypropylene, or a polyvinyl chloride resin is used. The water-insoluble pigment composition according to the present invention can be kneaded into the above-mentioned resin in a conventionally known manner.

### (Toner Applications)

The water-insoluble pigment composition according to the present invention can also be used for toner coloring applications. To obtain an electrostatic image development toner, a thermoplastic resin being solid at room temperature and having film forming properties, such as a polyester resin, a polyamide resin, a styrene resin, or an acrylic resin, is used as a dispersion resin.

The electrostatic image development toner produced using the water-insoluble pigment composition according to the present invention as a component can be used as a one-component color magnetic toner containing a magnetic substance in the toner (a color toner for magnetic one-component development), a non-magnetic one-component color toner containing no magnetic substance (a color toner for non-magnetic one-component development), or a color toner for a two-component color developer mixed with a carrier (a color toner for two-component development).

The one-component color magnetic toner can be formed from other additives, for example, a coloring agent, a binding resin, magnetic powder, a charge control agent (CCA), and a mold release agent, as is the case with a commonly used one-component color magnetic toner.

The amount of the water-insoluble pigment composition contained in the electrostatic charge image development toner is not particularly limited, but is preferably 0.5 to 25 parts by mass with respect to 100 parts by mass of the binding resin, and more preferably 4 to 10 parts by mass with respect to 100 parts by mass of the binding resin from the viewpoint of further enhancing the electrostatic properties of the coloring agent.

As the binding resin to be used for the electrostatic image development toner, any of the known and commonly used ones exemplified as the thermoplastic resin can be used, and any of synthetic resin, natural resin, natural rubber, synthetic rubber, synthetic wax, and the like that exhibit adhesive properties under the application of heat or pressure can be used.

### Examples

Hereinafter, the present invention will be described in more detail below with examples, but the scope of the present invention is not limited by these examples.

### (Example 1)

To a 3-L beaker, 20.0 g of hydroxyapatite (manufactured by FUJIFILM Wako Pure Chemical Corporation) and 1000 g of ion-exchanged water were added, and stirred at room temperature for 5 minutes by using a glass stirring blade connected to a three-one motor to prepare a water dispersion of hydroxyapatite. To a 500-mL beaker, 2.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare an annatto pigment solution. The annatto pigment solution was added to the water dispersion of hydroxyapatite and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and then the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 19.9 g of a powder (1). The composition ratio of the hydroxyapatite to the annatto pigment in the powder (1) is 91:9 in terms of the amount of addition. The resulting powder had an orange color similar to that of the annatto pigment.

To a 10-mL vial, 10 mg of the powder (1) obtained above, 1.0 g of ion-exchanged water, and a stir bar were added, and stirred with a magnetic stirrer for 5 minutes to prepare a liquid dispersion (1). When one drop of the liquid dispersion (1) was dropped onto a paper filter, it was observed that a portion of the paper filter onto which the liquid dispersion (1) was dropped colored circularly in orange, and then a colorless, transparent liquid spread concentrically. The orange-colored portion indicated the presence of the powder (1) that did not dissolve in water, whereas a portion in which the colorless, transparent liquid subsequently spread concentrically indicated the presence of water, hence it was confirmed that the powder (1) was water-insoluble.

### (Example 2)

To a 3-L beaker, 20.0 g of hydroxyapatite (manufactured by FUJIFILM Wako Pure Chemical Corporation) and 1000 g of ion-exchanged water were added, and stirred at room temperature for 5 minutes by using a glass stirring blade connected to a three-one motor to prepare a water dispersion of hydroxyapatite. To a 500-mL beaker, 6.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare an annatto pigment solution. The annatto pigment solution was added to the water dispersion of hydroxyapatite and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and then the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.3 g of powder (2).

The composition ratio of the hydroxyapatite to the annatto pigment in the powder (2) obtained above was 77:23 in terms of the amount of addition. The resulting powder had an orange color similar to that of the annatto pigment. To a 10-mL vial, 10 mg of the powder (2), 1.0 g of ion-exchanged water, and a stir bar were added and stirred with a magnetic stirrer for 5 minutes to prepare a liquid dispersion (2). When one drop of the liquid dispersion (2) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (2) that did not dissolve in water, whereas a portion in which the colorless, transparent liquid subsequently spread concentrically indicated the presence of water, hence it was confirmed that the powder (2) was water-insoluble.

### (Example 3)

To a 3-L beaker, 20.0 g of hydroxyapatite (manufactured by FUJIFILM Wako Pure Chemical Corporation) and 1000 g of ion-exchanged water were added, and stirred at room temperature for 5 minutes by using a glass stirring blade connected to a three-one motor to prepare a water dispersion of hydroxyapatite. To a 500-mL beaker, 2.0 g of sodium copper chlorophyllin (manufactured by FUJIFILM Wako Pure Chemical Corporation), 300 mL of ion-exchanged water, and a stir bar were added and stirred using a magnetic stirrer at room temperature for 15 minutes to prepare a pigment solution. The pigment solution was added to the water dispersion of hydroxyapatite and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and then the mixture was stirred at room temperature for 2 hours. When one drop of the resulting slurry was dropped onto a paper filter, it was observed that a portion of the paper filter onto which the slurry was dropped colored circularly in green, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 20.2 g of a powder (3).

The composition ratio of the hydroxyapatite to the sodium copper chlorophyllin in the powder (3) obtained above was 91:9 in terms of the amount of addition. The resulting powder had a green color similar to that of the sodium copper chlorophyllin. To a 10-mL vial, 10 mg of the powder (3), 1.0 g of ion-exchanged water, and a stir bar were added and stirred with a magnetic stirrer for 5 minutes to prepare a liquid dispersion (3). When one drop of the liquid dispersion (3) was dropped onto a paper filter, it was observed that a portion of the paper filter onto which the liquid dispersion (3) was dropped colored circularly in green, and then a colorless, transparent liquid spread concentrically. The green-colored portion indicated the presence of the powder (3) that did not dissolve in water, whereas a portion in which the colorless, transparent liquid subsequently spread concentrically indicated the presence of water, hence it was confirmed that the powder (3) was water-insoluble.

### (Example 4)

To a 3-L beaker, 20.0 g of organically-modified bentonite treated with quaternary ammonium (Moistnite-WO, manufactured by KUNIMINE INDUSTRIES **CO.,** LTD.) and 100 g of ethanol (Cica first grade (Extra pure), manufactured by KANTO CHEMICAL **CO.,** INC.) were added and sufficiently moistened. Next, 1000 g of ion-exchanged water was added, and the resulting solution was stirred at room temperature for 5 minutes by using a glass stirring blade connected to a three-one motor to prepare a dispersion of organically-modified bentonite. To a 500-mL beaker, 2.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare a pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.1 g of powder (4).

The composition ratio of the organically-modified bentonite to the annatto pigment in the powder (4) obtained above was 91:9 in terms of the amount of addition. The resulting powder had an orange color similar to that of the annatto pigment. To a 10-mL vial, 10 mg of the powder (4), 1.0 g of ion-exchanged water, and a stir bar were added and stirred with a magnetic stirrer for 5 minutes to prepare a liquid dispersion (4). When one drop of the liquid dispersion (4) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (4) that did not dissolve in water, whereas a portion in which the colorless, transparent liquid subsequently spread concentrically indicated the presence of water, hence it was confirmed that the powder (4) was water-insoluble.

### (Example 5)

To a 3-L beaker, 20.0 g of organically-modified bentonite treated with quaternary ammonium (Moistnite-WO, manufactured by KUNIMINE INDUSTRIES **CO.,** LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified bentonite. To a 500-mL beaker, 5.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare an annatto pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.5 g of powder (5).

The composition ratio of the organically-modified bentonite to the annatto pigment in the powder (5) obtained above was 80:20 in terms of the amount of addition. The resulting powder had an orange color similar to that of the annatto pigment. To a 10-mL vial, 10 mg of the powder (5), 1.0 g of ion-exchanged water, and a stir bar were added and stirred with a magnetic stirrer for 5 minutes to prepare a liquid dispersion (5). When one drop of the liquid dispersion (5) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (5) that did not dissolve in water, whereas a portion in which the colorless, transparent liquid subsequently spread concentrically indicated the presence of water, hence it was confirmed that the powder (5) was water-insoluble.

### (Example 6)

To a 3-L beaker, 20.0 g of organically-modified bentonite treated with quaternary ammonium (Moistnite-WO, manufactured by KUNIMINE INDUSTRIES CO., LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified bentonite. To a 500-mL beaker, 2.0 g of sodium copper chlorophyllin (manufactured by FUJIFILM Wako Pure Chemical Corporation), 300 mL of ion-exchanged water, and a stir bar were added and stirred using a magnetic stirrer at room temperature for 15 minutes to prepare a pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in green, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.1 g of powder (6).

The composition ratio of the organically-modified bentonite to the sodium copper chlorophyllin in the powder (6) obtained above was 91:9 in terms of the amount of addition. The resulting powder had a green color similar to that of the sodium copper chlorophyllin. To a 10-mL vial, 10 mg of the powder (6), 1.0 g of ion-exchanged water, and a stir bar were added and stirred with a magnetic stirrer for 5 minutes to prepare a liquid dispersion (6). When one drop of the liquid dispersion (6) was dropped onto a paper filter, it was observed that a portion of the paper filter onto which the liquid dispersion (3) was dropped colored circularly in green, and then a colorless, transparent liquid spread concentrically. The green-colored portion indicated the presence of the powder (6) that did not dissolve in water, whereas a portion in which the colorless, transparent liquid subsequently spread concentrically indicated the presence of water, hence it was confirmed that the powder (6) was water-insoluble.

### (Example 7)

To a 3-L beaker, 30.0 g of organically-modified smectite (Sumecton-SAN-P, manufactured by KUNIMINE INDUSTRIES CO., LTD.) treated with quaternary ammonium and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified smectite. To a 500-mL beaker, 3.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare a pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 29.8 g of powder (7).

The composition ratio of the organically-modified smectite to the annatto pigment in the powder (7) obtained above was 91:9 in terms of the amount of addition. The resulting powder had an orange color similar to that of the annatto pigment. To a 10-mL vial, 10 mg of the powder (7), 1.0 g of ion-exchanged water, and a stir bar were added and stirred with a magnetic stirrer for 5 minutes to prepare a liquid dispersion (7). When one drop of the liquid dispersion (7) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (7) that did not dissolve in water, whereas a portion in which the colorless, transparent liquid subsequently spread concentrically indicated the presence of water, hence it was confirmed that the powder (7) was water-insoluble.

### (Example 8)

To a 3-L beaker, 20.0 g of organically-modified bentonite treated with quaternary ammonium (Moistnite-WO, manufactured by KUNIMINE INDUSTRIES CO., LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified bentonite. To a 500-mL beaker, 6.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare an annatto pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 19.5 g of powder (8).

The composition ratio of the organically-modified bentonite to the annatto pigment in the powder (8) obtained above was 77:23 in terms of the amount of addition. The resulting powder had an orange color similar to that of the annatto pigment. To a 10-mL vial, 10 mg of the powder (8), 1.0 g of ion-exchanged water, and a stir bar were added and stirred with a magnetic stirrer for 5 minutes to prepare a liquid dispersion (8). When one drop of the liquid dispersion (8) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (8) that did not dissolve in water, whereas a portion in which the colorless, transparent liquid subsequently spread concentrically indicated the presence of water, hence it was confirmed that the powder (8) was water-insoluble.

### (Example 9)

To a 3-L beaker, 20.0 g of organically-modified bentonite treated with quaternary ammonium (Moistnite-WO, manufactured by KUNIMINE INDUSTRIES CO., LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified bentonite. To a 500-mL beaker, 1.0 g of SAFFLOWER Y1500 (manufactured by Daiwa Dyestuff Mfg. Co., Ltd., safflower yellow pigment: 85%, dextrin: 15%), 300 mL of ion-exchanged water, and a stir bar were added, and stirred using a magnetic stirrer at room temperature for 15 minutes to prepare a safflower yellow pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in yellow, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.7 g of powder (9) .

The composition ratio of the organically-modified bentonite to the safflower yellow pigment in the powder (9) obtained above was 96:4 in terms of the amount of addition. The resulting powder had a yellow color similar to that of the safflower yellow pigment. To a 10-mL vial, 10 mg of the powder (9), 1.0 g of ion-exchanged water, and a stir bar were added and stirred with a magnetic stirrer for 5 minutes to prepare a liquid dispersion (9). When one drop of the liquid dispersion (9) was dropped onto a paper filter, it was observed that a portion of the paper filter onto which the liquid dispersion (9) was dropped colored circularly in yellow, and then a colorless, transparent liquid spread concentrically. The yellow-colored portion indicated the presence of the powder (9) that did not dissolve in water, whereas a portion in which the colorless, transparent liquid subsequently spread concentrically indicated the presence of water, hence it was confirmed that the powder (9) was water-insoluble.

### (Example 10)

To a 3-L beaker, 20.0 g of organically-modified bentonite treated with quaternary ammonium (Moistnite-WO, manufactured by KUNIMINE INDUSTRIES CO., LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of organically-modified bentonite. To a 500-mL beaker, 2.0 g of Caramel I (manufactured by KANTO CHEMICAL CO., INC.), 300 mL of ion-exchanged water, and a stir bar were added and stirred using a magnetic stirrer at room temperature for 15 minutes to prepare a caramel pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL **CO.,** INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in brown, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.5 g of powder (10).

The composition ratio of the organically-modified bentonite to Caramel I in the powder (10) obtained above was 91:9 in terms of the amount of addition. The resulting powder had a brown color similar to that of the caramel pigment. To a 10-mL vial, 10 mg of the powder (10), 1.0 g of ion-exchanged water, and a stir bar were added and stirred with a magnetic stirrer for 5 minutes to prepare a liquid dispersion (10). When one drop of the liquid dispersion (10) was dropped onto a paper filter, it was observed that a portion of the paper filter onto which the liquid dispersion (10) was dropped colored circularly in brown, and then a colorless, transparent liquid spread concentrically. The brown-colored portion indicated the presence of the powder (10) that did not dissolve in water, whereas a portion in which the colorless, transparent liquid subsequently spread concentrically indicated the presence of water, hence it was confirmed that the powder (10) was water-insoluble.

### (Example 11)

To a 3-L beaker, 20.0 g of hydroxyapatite (manufactured by FUJIFILM Wako Pure Chemical Corporation) and 1000 g of ion-exchanged water were added, and stirred at room temperature for 5 minutes by using a glass stirring blade connected to a three-one motor to prepare a water dispersion of hydroxyapatite. To a 500-mL beaker, 2.0 g of Caramel I (manufactured by KANTO CHEMICAL CO., INC.), 300 mL of ion-exchanged water, and a stir bar were added and stirred using a magnetic stirrer at room temperature for 15 minutes to prepare a pigment solution. The pigment solution was added to the water dispersion of hydroxyapatite and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and then the mixture was stirred at room temperature for 2 hours. When one drop of the resulting slurry was dropped onto a paper filter, it was observed that a portion of the paper filter onto which the slurry was dropped colored circularly in brown, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 19.0 g of powder (11).

The composition ratio of the hydroxyapatite to Caramel I in the powder (11) obtained above was 91:9 in terms of the amount of addition. The resulting powder had a brown color similar to that of Caramel I. To a 11-mL vial, 10 mg of the powder (11), 1.0 g of ion-exchanged water, and a stir bar were added and stirred with a magnetic stirrer for 5 minutes to prepare a liquid dispersion (10). When one drop of the liquid dispersion (11) was dropped onto a paper filter, it was observed that a portion of the paper filter onto which the liquid dispersion (10) was dropped colored circularly in brown, and then a colorless, transparent liquid spread concentrically. The brown-colored portion indicated the presence of the powder (11) that did not dissolve in water, whereas a portion in which the colorless, transparent liquid subsequently spread concentrically indicated the presence of water, hence it was confirmed that the powder (11) was water-insoluble.

### (Example 12)

To a 3-L beaker, 20.0 g of smectite (Sumecton-SWN, manufactured by KUNIMINE INDUSTRIES CO., LTD.) and 100 g of ethanol (Cica first grade (Extra pure), manufactured by KANTO CHEMICAL CO., INC.) were added and sufficiently moistened. Next, 1000 g of ion-exchanged water was added, and the resulting solution was stirred at room temperature for 5 minutes by using a glass stirring blade connected to a three-one motor to prepare a water dispersion of smectite. To a 500-mL beaker, 2.0 g of an annatto pigment (manufactured by KANTO CHEMICAL CO., INC.), 300 mL of ion-exchanged water, and a stir bar were added, and stirred using a magnetic stirrer at room temperature for 15 minutes to prepare a pigment solution. The pigment solution was added to the water dispersion and stirred at room temperature for 15 minutes. Subsequently, dilute hydrochloric acid adjusted by diluting hydrochloric acid (Cica first grade (Extra pure), manufactured by KANTO CHEMICAL CO., INC.) with ion-exchanged water by 10-fold dilution was slowly added dropwise with a dropper to adjust the pH to 4.0, and the mixture was stirred at room temperature for 2 hours. When one drop of the resulting slurry was dropped onto a paper filter, it was observed that a portion of the paper filter onto which the slurry was dropped colored circularly in orange, and then a colorless, transparent liquid spread concentrically, hence it was confirmed that the pigment became water-insoluble. Next, the slurry was filtered through a Nutsche filter and washed with 2000 g of ion-exchanged water, and the resulting solid was dried in a vacuum dryer (740 mmHg) at 30°C for 14 hours, and then pulverized with a juicer to obtain 18.8 g of powder (12).

The composition ratio of the smectite to the annatto pigment in the powder (12) obtained above was 91:9 in terms of the amount of addition. The resulting powder had an orange color similar to that of the annatto pigment. To a 12-mL vial, 10 mg of the powder (12), 1.0 g of ion-exchanged water, and a stir bar were added and stirred with a magnetic stirrer for 5 minutes to prepare a liquid dispersion (10). When one drop of the liquid dispersion (12) was dropped onto a paper filter, it was observed that a portion of the paper filter onto which the liquid dispersion (2) was dropped colored circularly in orange, and then a colorless, transparent liquid spread concentrically. The orange-colored portion indicated the presence of the powder (12) that did not dissolve in water, whereas a portion in which the colorless, transparent liquid subsequently spread concentrically indicated the presence of water, hence it was confirmed that the powder (12) was water-insoluble.

### (Comparative Example 1)

To a 10-mL vial, 10 mg of an annatto pigment (manufactured by KANTO CHEMICAL CO., INC.) and 1.0 g of ion-exchanged water were added, and then a stir bar was added thereto and stirred for 5 minutes to prepare a liquid dispersion (13). When one drop of the liquid dispersion (13) was dropped onto a paper filter, it was observed that an orange-colored liquid uniformly spread concentrically around a portion of the paper filter onto which the liquid dispersion (13) was dropped.

### (Comparative Example 2)

To a 10-mL vial, 10 mg of sodium copper chlorophyllin (manufactured by FUJIFILM Wako Pure Chemical Corporation) and 1.0 g of ion-exchanged water were added, and then a stir bar was added thereto and stirred for 5 minutes to prepare a liquid dispersion (14). When one drop of the liquid dispersion (14) was dropped onto a paper filter, it was observed that a green-colored liquid uniformly spread concentrically around a portion of the paper filter onto which the liquid dispersion (14) was dropped.

### (Comparative Example 3)

To a 10-mL vial, 0.9 mg of an annatto pigment (manufactured by KANTO CHEMICAL CO., INC.), 9.1 mg of hydroxyapatite (FL-HAP(SC), manufactured by TAIHEI CHEMICAL INDUSTRIAL CO., LTD.), and 1.0 g of ion-exchanged water were added, and then a stir bar was added thereto and stirred for 5 minutes to prepare a liquid dispersion (15). When one drop of the liquid dispersion (15) was dropped onto a paper filter, it was observed that an orange-colored liquid uniformly spread concentrically around a portion of the paper filter onto which the liquid dispersion (13) was dropped.

### (Comparative Example 4)

To a 10-mL vial, 2.3 mg of an annatto pigment (manufactured by KANTO CHEMICAL CO., INC.), 7.7 mg of hydroxyapatite (FL-HAP(SC), manufactured by TAIHEI CHEMICAL INDUSTRIAL CO., LTD.), and 1.0 g of ion-exchanged water were added, and then a stir bar was added thereto and stirred for 5 minutes to prepare a liquid dispersion (16). When one drop of the liquid dispersion (16) was dropped onto a paper filter, it was observed that an orange-colored liquid uniformly spread concentrically around a portion of the paper filter onto which the liquid dispersion (13) was dropped.

### (Comparative Example 5)

To a 10-mL vial, 0.9 mg of sodium copper chlorophyllin (manufactured by FUJIFILM Wako Pure Chemical Corporation), 9.1 mg of hydroxyapatite (FL-HAP(SC), manufactured by TAIHEI CHEMICAL INDUSTRIAL CO., LTD.), and 1.0 g of ion-exchanged water were added, and then a stir bar was added thereto and stirred for 5 minutes to prepare a liquid dispersion (17). When one drop of the liquid dispersion (17) was dropped onto a paper filter, it was observed that a green-colored liquid uniformly spread concentrically around a portion of the paper filter onto which the liquid dispersion (14) was dropped.

### (Comparative Example 6)

To a 10-mL vial, 0.9 mg of an annatto pigment (manufactured by KANTO CHEMICAL CO., INC.), 9.1 mg of organically-modified bentonite treated with quaternary ammonium (Moistnite-WO, manufactured by KUNIMINE INDUSTRIES CO., LTD.), and 1.0 g of ion-exchanged water were added, and then a stir bar was added thereto and stirred for 5 minutes to prepare a liquid dispersion (18). When one drop of the liquid dispersion (18) was dropped onto a paper filter, it was observed that an orange-colored liquid uniformly spread concentrically around a portion of the paper filter onto which the liquid dispersion (13) was dropped.

### (Comparative Example 7)

To a 10-mL vial, 2.0 mg of an annatto pigment (manufactured by KANTO CHEMICAL CO., INC.), 8.0 mg of organically-modified bentonite treated with quaternary ammonium (Moistnite-WO, manufactured by KUNIMINE INDUSTRIES CO., LTD.), and 1.0 g of ion-exchanged water were added, and then a stirrer was added thereto and stirred for 5 minutes to prepare a liquid dispersion (19). When one drop of the liquid dispersion (19) was dropped onto a paper filter, it was observed that an orange-colored liquid uniformly spread concentrically around a portion of the paper filter onto which the liquid dispersion (13) was dropped.

### (Comparative Example 8)

To a 10-mL vial, 0.9 mg of sodium copper chlorophyllin (manufactured by FUJIFILM Wako Pure Chemical Corporation), 9.1 mg of organically-modified bentonite treated with quaternary ammonium (Moistnite-WO, manufactured by KUNIMINE INDUSTRIES CO., LTD.), and 1.0 g of ion-exchanged water were added, and then a stir bar was added thereto and stirred for 5 minutes to prepare a liquid dispersion (20). When one drop of the liquid dispersion (20) was dropped onto a paper filter, it was observed that a green-colored liquid uniformly spread concentrically around a portion of the paper filter onto which the liquid dispersion (14) was dropped.

### (Comparative Example 9)

To a 10-mL vial, 0.9 mg of an annatto pigment (manufactured by KANTO CHEMICAL CO., INC.), 9.1 mg of organically-modified smectite treated with quaternary ammonium (Sumecton-SAN-P, manufactured by KUNIMINE INDUSTRIES CO., LTD.), and 1.0 g of ion-exchanged water were added, and then a stir bar was added thereto and stirred for 5 minutes to prepare a liquid dispersion (21). When one drop of the liquid dispersion (21) was dropped onto a paper filter, it was observed that an orange-colored liquid uniformly spread concentrically around a portion of the paper filter onto which the liquid dispersion (13) was dropped.

### (Comparative Example 10)

To a 10-mL vial, 2.3 mg of an annatto pigment (manufactured by KANTO CHEMICAL .CO., INC.), 7.7 mg of organically-modified bentonite treated with quaternary ammonium (Moistnite-WO, manufactured by KUNIMINE INDUSTRIES CO., LTD.), and 1.0 g of ion-exchanged water were added, and then a stir bar was added thereto and stirred for 5 minutes to prepare a liquid dispersion (22). When one drop of the liquid dispersion (22) was dropped onto a paper filter, it was observed that an orange-colored liquid uniformly spread concentrically around a portion of the paper filter onto which the liquid dispersion (13) was dropped.

### (Comparative Example 11)

To a 10-mL vial, 10 mg of SAFFLOWER Y1500 (manufactured by Daiwa Dyestuff Mfg. Co., Ltd., safflower yellow pigment: 85%, dextrin: 15%) and 1.0 g of ion-exchanged water were added, and then a stir bar was added thereto and stirred for 5 minutes to prepare a liquid dispersion (23). When one drop of the liquid dispersion (23) was dropped onto a paper filter, it was observed that a yellow-colored liquid uniformly spread concentrically around a portion of the paper filter onto which the liquid dispersion (23) was dropped.

### (Comparative Example 12)

To a 10-mL vial, 10 mg of a pigment Caramel I (manufactured by KANTO CHEMICAL CO., INC.) and 1.0 g of ion-exchanged water were added, and then a stir bar was added thereto and stirred for 5 minutes to prepare a liquid dispersion (24). When one drop of the liquid dispersion (24) was dropped onto a paper filter, it was observed that a brown-colored liquid uniformly spread concentrically around a portion of the paper filter onto which the liquid dispersion (24) was dropped.

### (Comparative Example 13)

To a 10-mL vial, 0.5 mg of SAFFLOWER Y1500 (manufactured by Daiwa Dyestuff Mfg. Co., Ltd., safflower yellow pigment: 85%, dextrin: 15%), 9.5 mg of organically-modified bentonite treated with quaternary ammonium (Moistnite-WO, manufactured by KUNIMINE INDUSTRIES CO., LTD.), and 1.0 g of ion-exchanged water were added, and then a stir bar was added thereto and stirred for 5 minutes to prepare a liquid dispersion (25). When one drop of the liquid dispersion (25) was dropped onto a paper filter, it was observed that an orange-colored liquid uniformly spread concentrically around a portion of the paper filter onto which the liquid dispersion (13) was dropped.

### (Comparative Example 14)

To a 10-mL vial, 2.3 mg of Caramel I (manufactured by KANTO CHEMICAL CO., INC.), 7.7 mg of organically-modified bentonite treated with quaternary ammonium (Moistnite-WO, manufactured by KUNIMINE INDUSTRIES CO., LTD.), and 1.0 g of ion-exchanged water were added, and then a stir bar was added thereto and stirred for 5 minutes to prepare a liquid dispersion (26). When one drop of the liquid dispersion (26) was dropped onto a paper filter, it was observed that a brown-colored liquid uniformly spread concentrically around a portion of the paper filter onto which the liquid dispersion (24) was dropped.

### (Comparative Example 15)

To a 10-mL vial, 0.9 mg of a pigment Caramel I (manufactured by KANTO CHEMICAL CO., INC.), 9.1 mg of hydroxyapatite (FL-HAP(SC), manufactured by TAIHEI CHEMICAL INDUSTRIAL CO., LTD.), and 1.0 g of ion-exchanged water were added, and then a stir bar was added thereto and stirred for 5 minutes to prepare a liquid dispersion (27). When one drop of the liquid dispersion (27) was dropped onto a paper filter, it was observed that an orange-colored liquid uniformly spread concentrically around a portion of the paper filter onto which the liquid dispersion (13) was dropped.

### (Comparative Example 16)

To a 10-mL vial, 0.9 mg of an annatto pigment (manufactured by KANTO CHEMICAL CO., INC.), 9.1 mg of untreated smectite (Sumecton-SWN, manufactured by KUNIMINE INDUSTRIES CO., LTD.), and 1.0 g of ion-exchanged water were added, and then a stir bar was added thereto and stirred for 5 minutes to prepare a liquid dispersion (28). When one drop of the liquid dispersion (28) was dropped onto a paper filter, it was observed that an orange-colored liquid uniformly spread concentrically around a portion of the paper filter onto which the liquid dispersion (13) was dropped.

Cosmetics, such as a lipstick cosmetic, an eye cosmetic, and a nail cosmetic, blended with the water-insoluble pigment composition according to the present invention have higher performance, compared to cosmetics blended with natural pigments as they are.

### (Evaluation Example)

### (Production and Evaluation of Lipstick Cosmetics)

A balm cream base (manufactured by Orangeflower) and castor oil (manufactured by Orangeflower) were weighed out and put into a pudding cup, and mixed while heated in a hot water bath at a temperature of 70°C to 80°C. One of the powders prepared in Examples and natural pigments was added to the mixture, and mixed while heated. The resulting mixed solution was filled into a silicon mold with a ring and left standing for 10 minutes, then cooled in a refrigerator at 10°C for approximately 10 minutes. A portion of the resulting lipstick cosmetic that protruded from the ring was scraped off with a spatula, and the ring was removed, then a cylinder for lipsticks was fitted in. A lower part of the silicon mold was pinched to let a small amount of air in and the cylinder was moved down little by little so that the lipstick cosmetic was accommodated in the cylinder, whereby a lipstick cosmetic was produced. For lipstick cosmetics, the powders prepared in Examples and the natural pigments were used as colorants, respectively. As the natural pigments, an annatto pigment (manufactured by KANTO CHEMICAL CO., INC.), sodium copper chlorophyllin (manufactured by FUJIFILM Wako Pure Chemical Corporation), and SAFFLOWER Y1500 (manufactured by Daiwa Dyestuff Mfg. Co., Ltd., safflower yellow pigment: 85%, dextrin: 15%) were used. Note that the amount of each of the colorants added was based on the parts by mass of the pigment in the formulation. The produced lipstick cosmetics were summarized in Table 1.

**[Table 1]**

| Evaluation Ex./ Comp. Evaluation Ex. | Colorant | Pigment in Powder | Carrier Substance in Powder | Addition Amount (g) | | | |
|---|---|---|---|---|---|---|---|
| | | | | Colorant (Pigment Amount) | Cream Base | Castor Oil | Total |
| Evaluation Ex. 1 | Powder 2 | Annatto pigment | Hydroxyapatite | 0.3 (0.07) | 5.15 | 0.3 | 5.75 |
| Evaluation Ex. 2 | Powder 3 | Sodium copper chlorophyllin | Hydroxyapatite | 0.3 (0.03) | 5.15 | 0.3 | 5.75 |
| Evaluation Ex. 3 | Powder 4 | Annatto pigment | Organically-modified bentonite | 0.3 (0.03) | 5.15 | 0.3 | 5.75 |
| Evaluation Ex. 4 | Powder 6 | Sodium copper chlorophyllin | Organically-modified bentonite | 0.3 (0.03) | 5.15 | 0.3 | 5.75 |
| Evaluation Ex. 5 | Powder 9 | Safflower yellow pigment | Organically-modified bentonite | 0.3 (0.015) | 5.15 | 0.3 | 5.75 |
| Comp. Evaluation Ex. 1 | Annatto pigment | - | - | 0.07 (0.07) | 5.38 | 0.3 | 5.75 |
| Comp. Evaluation Ex. 2 | Annatto pigment | - | - | 0.03 (0.03) | 5.42 | 0.3 | 5.75 |
| Comp. Evaluation Ex. 3 | Sodium copper chlorophyllin | - | - | 0.03 (0.03) | 5.42 | 0.3 | 5.75 |
| Comp. Evaluation Ex. 4 | SAFFLOWER Y1500 | - | - | 0.018 (0.015) | 5.43 | 0.3 | 5.75 |

The water resistance, coarse-grained appearance, feel, color irregularity, and makeup durability of each of the lipstick cosmetics were evaluated. The water resistance was evaluated by applying the lipstick cosmetic to a paper filter and visually checking the degree of bleeding when 1 mL of water was dropped onto a colored portion by a dropper. The coarse-grained appearance was evaluated by visually checking the lipstick cosmetic. The feel and the color irregularity were evaluated by applying the lipstick cosmetic to a wrist. The makeup durability was evaluated by visually checking how much the color faded after the lipstick cosmetic applied to the wrist was rubbed three times with tissue paper. Evaluation results were summarized in Table 2. The water resistance was rated as "○" when bleeding was absent, and rated as "x" when bleeding was present. The coarse-grained appearance was rated as "o" when no coarse grain was present, and rated as "x" when coarse grains were present. The feel was rated as "o" when the feel was good, and the feel was rated as "x" when the feel was poor. The color irregularity was rated as "o" when irregularities in coloring were absent, and rated as "x" when irregularities in coloring were present. The makeup durability was rated as "o" when color fading was absent, and rated as "x" when color fading was present.

**[Table 2]**

| Evaluation Ex./ Comp. Evaluation Ex. | Pigment in Powder | Carrier Substance in Powder | Carrier Substance in Powder | Evaluation Result | | | | |
|---|---|---|---|---|---|---|---|---|
| | | | | Water Resistance | Coarse-grained Appearance | Feel | Color Irregularity | Makeup Durability |
| Evaluation Ex. 1 | Powder 2 | Annatto pigment | Hydroxyapatite | ○ | ○ | ○ | ○ | ○ |
| Evaluation Ex. 2 | Powder 3 | Sodium copper chlorophyllin | Hydroxyapatite | ○ | ○ | ○ | ○ | ○ |
| Evaluation Ex. 3 | Powder 4 | Annatto pigment | Organically-modified bentonite | ○ | ○ | ○ | ○ | ○ |
| Evaluation Ex. 4 | Powder 6 | Sodium copper chlorophyllin | Organically-modified bentonite | ○ | ○ | ○ | ○ | ○ |
| Evaluation Ex. 5 | Powder 9 | Safflower yellow pigment | Organically-modified bentonite | ○ | ○ | ○ | ○ | ○ |
| Comp. Evaluation Ex. 1 | Annatto pigment | - | - | × | × | × | × | × |
| Comp. Evaluation Ex. 2 | Annatto pigment | - | - | × | × | × | × | × |
| Comp. Evaluation Ex. 3 | Sodium copper chlorophyllin | - | - | × | × | × | × | × |
| Comp. Evaluation Ex. 4 | SAFFLOWER Y1500 | - | - | × | × | × | × | × |

As revealed in Table 2, the lipstick cosmetics including the water-insoluble pigment composition according to the present invention were superior in waterinsolubility, coarse-grained appearance, feel, color irregularity, and makeup durability, compared to the lipstick cosmetics including the natural pigments as they were.

### (Production and Evaluation of Eye Cosmetics)

14.1 g of talc (manufactured by YAMAGUCHI MICA CO., LTD.), 0.02 g of methylparaben (manufactured by Maruzen Chemicals Co., Ltd.), 0.02 g of propylparaben (manufactured by Maruzen Chemicals Co., Ltd.), and 1.88 g of trihydroxystearin (manufactured by Matsumoto Trading Co., Ltd.) were weighed out and put into a coffee grinder, and stirring for 10 seconds was performed three times to prepare a dispersion base. The dispersion base and one of the powders prepared in Examples and natural pigments were added to a coffee grinder, and stirring for 5 seconds was performed twice to prepare an eye cosmetic powder. The eye cosmetic powder was placed in a compact-pan and pressed to produce an eye cosmetic. For eye cosmetics, the powders prepared in Examples were used as colorants, respectively. Eye cosmetics each including one of an annatto pigment (manufactured by KANTO CHEMICAL CO., INC.), sodium copper chlorophyllin (manufactured by FUJIFILM Wako Pure Chemical Corporation), and SAFFLOWER Y1500 (manufactured by Daiwa Dyestuff Mfg. Co., Ltd., safflower yellow pigment: 85%, dextrin: 15%) were produced for comparison. Note that the amount of each of the colorants added was based on the parts by mass of the pigment in the formulation. The produced eye cosmetics were summarized in Table 3.

**[Table 3]**

| Evaluation Ex./ Comp. Evaluation Ex. | Colorant | Pigment in Powder | Carrier Substance in Powder | Addition Amount (g) | | |
|---|---|---|---|---|---|---|
| | | | | Colorant (Pigment) | Dispersion Base | Total |
| Evaluation Ex. 6 | Powder 2 | Annatto pigment | Hydroxyapatite | 0.75 (0.17) | 4.25 | 5.0 |
| Evaluation Ex. 7 | Powder 3 | Sodium copper chlorophyllin | Hydroxyapatite | 0.75 (0.07) | 4.25 | 5.0 |
| Evaluation Ex. 8 | Powder 4 | Annatto pigment | Organically-modified bentonite | 0.75 (0.07) | 4.25 | 5.0 |
| Evaluation Ex. 9 | Powder 6 | Sodium copper chlorophyllin | Organically-modified bentonite | 0.75 (0.07) | 4.25 | 5.0 |
| Evaluation Ex. 10 | Powder 8 | Annatto pigment | Organically-modified bentonite | 0.75 (0.17) | 4.25 | 5.0 |
| Evaluation Ex. 11 | Powder 9 | Safflower yellow pigment | Organically-modified bentonite | 0.75 (0.036) | 4.25 | 5.0 |
| Comp. Evaluation Ex. 5 | Annatto pigment | - | - | 0.17 (0.17) | 4.83 | 5.0 |
| Comp. Evaluation Ex. 6 | Annatto pigment | - | - | 0.07 (0.07) | 4.93 | 5.0 |
| Comp. Evaluation Ex. 7 | Sodium copper chlorophyllin | - | - | 0.07 (0.07) | 4.93 | 5.0 |
| Comp. Evaluation Ex. 8 | Safflower yellow pigment | - | - | 0.042 (0.036) | 4.96 | 5.0 |

The water resistance, roughness, color irregularity, and makeup durability of each of the eye cosmetics were evaluated. The water resistance was evaluated by applying the eye cosmetic to a paper filter and visually checking the degree of bleeding when 0.5 mL of water was dropped onto a colored portion by using a dropper. The roughness and the color irregularity were evaluated by applying the eye cosmetic to a wrist. The makeup durability was evaluated by checking how much the color remained after the eye cosmetic applied to the wrist was rubbed three times. Evaluation results were summarized in Table 4. The water resistance was rated as "o" when bleeding was absent, and rated as "x" when bleeding was present. The roughness was rated as "o" when roughness was absent, and rated as "x" when roughness was present. The color irregularity was rated as "o" when irregularities in coloring were absent, and rated as "x" when irregularities in coloring were present. The makeup durability was rated as "o" when color fading was absent, and rated as "x" when color fading was present.

**[Table 4]**

| Evaluation Ex./ Comp. Evaluation Ex. | Colorant | Pigment in Powder | Carrier Substance in Powder | Evaluation Item | | | |
|---|---|---|---|---|---|---|---|
| | | | | Water Resistance | Roughness | Color Irregularity | Makeup Durability |
| Evaluation Ex. 6 | Powder 2 | Annatto pigment | Hydroxyapatite | ○ | ○ | ○ | ○ |
| Evaluation Ex. 7 | Powder 3 | Sodium copper chlorophyllin | Hydroxyapatite | ○ | ○ | ○ | ○ |
| Evaluation Ex. 8 | Powder 4 | Annatto pigment | Organically-modified bentonite | ○ | ○ | ○ | ○ |
| Evaluation Ex. 9 | Powder 6 | Sodium copper chlorophyllin | Organically-modified bentonite | ○ | ○ | ○ | ○ |
| Evaluation Ex. 10 | Powder 8 | Annatto pigment | Organically-modified bentonite | ○ | ○ | ○ | ○ |
| Evaluation Ex. 11 | Powder 9 | Safflower yellow pigment | Organically-modified bentonite | ○ | ○ | ○ | ○ |
| Comp. Evaluation Ex. 5 | Annatto pigment | - | - | × | × | × | × |
| Comp. Evaluation Ex. 6 | Annatto pigment | - | - | × | × | × | x |
| Comp. Evaluation Ex. 7 | Sodium copper chlorophyllin | - | - | × | × | × | × |
| Comp. Evaluation Ex. 8 | Safflower yellow pigment | - | - | × | × | × | × |

As revealed in Table 4, the eye cosmetics including the water-insoluble pigment composition according to the present invention were superior in water resistance, roughness, color irregularity, and makeup durability, compared to the eye cosmetics including the natural pigments as they were.

### (Production and Evaluation of Nail Cosmetics)

To a 20-mL plastic bottle, 1.7 g of any one of the powders prepared in Examples and natural pigments and Nailholic base coating (color: SP030, manufactured by KOSÉ Corporation) were added and mixed with a dropper. The Nailholic base coating was additionally added to produce a nail cosmetic. For nail cosmetics, the powders prepared in Examples were used as colorants, respectively. Nail cosmetics including each including one of an annatto pigment (manufactured by KANTO CHEMICAL CO., INC.), sodium copper chlorophyllin (manufactured by FUJIFILM Wako Pure Chemical Corporation), and SAFFLOWER Y1500 (manufactured by Daiwa Dyestuff Mfg. Co., Ltd., safflower yellow pigment: 85%, dextrin: 15%) were produced for comparison. Note that the amount of each of the colorants and the Nailholic base coatings added was based on the parts by mass of the pigment in the formulation. The produced nail cosmetics were summarized in Table 5.

**[Table 5]**

| Evaluation Ex./ Comp. Evaluation Ex. | Colorant | Pigment in Powder | Carrier Substance in Powder | Addition Amount (g) | | |
|---|---|---|---|---|---|---|
| | | | | Colorant (Pigment) | Nailholic Base Coating | Total |
| Evaluation Ex. 12 | Powder 3 | Sodium copper chlorophyllin | Hydroxyapatite | 0.4 (0.09) | 4.60 | 5.0 |
| Evaluation Ex. 13 | Powder 4 | Annatto pigment | Organically-modified bentonite | 0.4 (0.09) | 4.60 | 5.0 |
| Evaluation Ex. 14 | Powder 6 | Sodium copper chlorophyllin | Organically-modified bentonite | 0.4 (0.09) | 4.60 | 5.0 |
| Evaluation Ex. 15 | Powder 9 | Safflower yellow pigment | Organically-modified bentonite | 0.4 (0.02) | 4.60 | 5.0 |
| Comp. Evaluation Ex. 9 | Annatto pigment | - | - | 0.09 | 4.91 | 5.0 |
| Comp. Evaluation Ex. 10 | Sodium copper chlorophyllin | - | - | 0.04 | 4.96 | 5.0 |
| Comp. Evaluation Ex. 11 | SAFFLOWER Y1500 | - | - | 0.023 (0.02) | 4.98 | 5.0 |

The water resistance, the unevenness of a coated surface, and the color irregularity of each of the nail cosmetics were evaluated. The water resistance was visually checked by applying the nail cosmetic to a paper filter and visually checking the degree of bleeding when 1 mL of water was dropped onto a colored portion by a dropper. The color irregularity and the unevenness of the coated surface were evaluated by applying the nail cosmetic to a nail tip, drying the nail cosmetic, and then checking the appearance and feel of the coated surface. Evaluation results were summarized in Table 6. The water resistance was rated as "o" when bleeding was absent, and rated as "x" when bleeding was present. The unevenness of the coated surface was rated as "o" when unevenness was absent, and rated as "x" when unevenness was present. The color irregularity was rated as "o" when irregularities in coloring were absent, and rated as "x" when irregularities in coloring were present.

**[Table 6]**

| Evaluation Ex./ Comp. Evaluation Ex. | Colorant | Pigment in Powder | Carrier Substance in Powder | Evaluation Item | | |
|---|---|---|---|---|---|---|
| | | | | Water Resistance | Unevenness of Coated Surface | Color Irregularity |
| Evaluation Ex. 12 | Powder 3 | Sodium copper chlorophyllin | Hydroxyapatite | ○ | ○ | ○ |
| Evaluation Ex. 13 | Powder 4 | Annatto pigment | Organically-modified bentonite | ○ | ○ | ○ |
| Evaluation Ex. 14 | Powder 6 | Sodium copper chlorophyllin | Organically-modified bentonite | ○ | ○ | ○ |
| Evaluation Ex. 15 | Powder 9 | Safflower yellow pigment | Organically-modified bentonite | ○ | ○ | ○ |
| Comp. Evaluation Ex. 9 | Annatto pigment | - | - | × | × | × |
| Comp. Evaluation Ex. 10 | Sodium copper chlorophyllin | - | - | × | × | × |
| Comp. Evaluation Ex. 11 | SAFFLOWER Y1500 | - | - | × | × | × |

As revealed in Table 6, the nail cosmetics including the water-insoluble pigment compositions according to the present invention were superior in water resistance, unevenness of the coated surface, and color irregularity, compared to the nail cosmetics using the natural pigments as they were.

## Claims

1. A water-insoluble pigment composition, produced by compositing a natural pigment with at least one selected from hydroxyapatite and a clay mineral.

2. The water-insoluble pigment composition according to claim 1, wherein a composition mass ratio of the natural pigment to the at least one selected from the hydroxyapatite and the clay mineral is the natural pigment : the hydroxyapatite or the clay minerals = 0.1:99.9 to 90:10.

3. The water-insoluble pigment composition according to claim 1 or 2, wherein the clay mineral is at least one selected from bentonite, hectorite, and smectite.

4. The water-insoluble pigment composition according to claim 1 or 2, wherein the clay mineral is at least one selected from organically-modified bentonite, organically-modified hectorite, and organically-modified smectite.

5. The water-insoluble pigment composition according to claim 1 or 2, wherein the natural pigment is at least one selected from a carotenoid pigment, a porphyrin pigment, a flavonoid pigment, and a pigment protein.

6. The water-insoluble pigment composition according to claim 1 or 2, wherein the natural pigment is at least one selected from an annatto pigment, a chlorophyll pigment, a safflower yellow pigment, and a phycocyanin pigment.

7. A food, a cosmetic, a lipstick cosmetic, an eye cosmetic, a nail cosmetic, a base makeup cosmetic, a pharmaceutical, a coating material for agricultural chemicals, a printing marker, a stationery product, a writing tool, a printing ink, an inkjet ink, a metal ink, a paint, a plastic coloring agent, a color toner, a fluorescent labeling agent, a fluorescent probe, or a chemical sensor, comprising the water-insoluble pigment composition according to claim 1 or 2.
